# FASCICULE DE BREVET EUROPEEN

(11) **EP 0 398 782 B1**
(45) Date de publication et mention de la délivrance du brevet: **14.09.1994**
(21) Numéro de dépôt: 90401222.6
(22) Date de dépôt: 09.05.1990
(51) Int. Cl.: C07C 255/40, C07C 255/17, C07C 255/31, C07C 253/14

(54) **Procédé de préparation de cyanures d'acyles en milieu anhydre**
Verfahren zur Herstellung von Acylcyaniden in wasserfreien Medien
Process for the preparation of acyl cyanides in anhydrous media

(30) Priorité: 19.05.1989 FR 8906563
(43) Date de publication de la demande: 22.11.1990
(73) Titulaire: ELF ATOCHEM S.A., 92800 Puteaux (FR)
(72) Inventeur: Devic, Michel, F-69110 Saint Foy Les Lyon (FR); Tellier, Pierre, F-69110 Saint Foy Les Lyon (FR)

(56) Documents cités:
- EP-A- 0 038 987
- FR-A- 2 346 323
- FR-A- 2 353 524
- FR-A- 2 364 894

## Description

La présente invention concerne un procédé de synthèse de cyanures d'acyle en milieu anhydre. Ce procédé consiste à faire réagir des halogènures d'acide avec un cyanure alcalin. Les cyanures d'acyle sont des intermédiaires de synthèse organique par exemple, des herbicides.

Le brevet FR 2 353 524 décrit une synthèse de cyanure de benzoyle C₆H₅COCN par réaction du chlorure de benzoyle avec du cyanure de sodium en excès molaire en présence de nitrile d'acide carboxylique et de cyanure de cuivre. Le brevet FR 2 346 323 décrit une réaction similaire mais beaucoup plus générale puisqu'elle s'applique à toute une famille de cyanures d'acyle et consiste à faire réagir du cyanure de sodium avec un halogénure d'acide en excès en présence de cyanure de cuivre ou de zinc. Ces procédés ont l'inconvénient de nécessiter la présence de métaux lourds et obligent donc à des traitements compliqués pour éviter leur présence dans les effluents On trouve dans TETRAHEDRON LETTERS (Pergamon Press) n^{o} 26, pages 2275-2278 (1974) un procédé limité à la synthèse du cyanure de benzoyle par action du cyanure de sodium sur le chlorure de benzoyle en solution dans le chlorure de méthylène et en présence de bromure de tétrabutylammonium, le rendement en C₆H₅COCN sur C₆H₅COCl ne dépasse pas 60 %. Le brevet FR 2 364 894 décrit la synthèse de C₆H₅COCN par action de C₆H₅COCl sur le NaCN dans un solvant en présence d'anhydride benzoïque (C₆H₅CO-O-CO-C₆H₅) ou de produits qui peuvent générer de l'anhydride benzoïque dans les conditions de la réaction.

La quantité d'anhydride benzoïque préférée est comprise entre 0,03 et 0,1 mole par mole de chlorure de benzoyle. Cette présence d'anhydride benzoïque complique la récupération du cyanure de benzoyle. On a maintenant trouvé un procédé qui permet de convertir du chlorure de benzoyle en cyanure de benzoyle et qui ne sous-produit pas d'anhydride benzoïque.

La présente invention est donc un procédé de synthèse ce cyanures d'acyle de formule (I) : 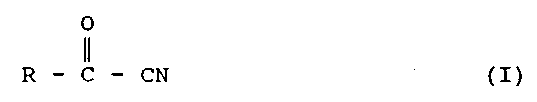
dans laquelle R est soit un radical alkyle ayant de 1 à 8 atomes de carbone, soit un radical cycloalkyle ayant de 3 à 12 atomes de carbone, soit un radical aryle, soit un reste hétérocyclique pouvant être condensé avec un noyau benzénique, tous ces radicaux R étant éventuellement substitués, consistant à faire réagir des halogénures d'acide de formule (II) : 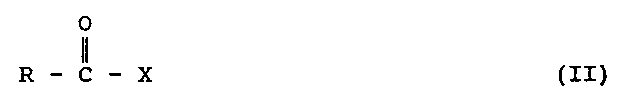
dans laquelle R a la définition précédente et X désigne Cl, Br, avec des cyanures alcalins, caractérisé en ce que la réaction a lieu :
a) en milieu solvant anhydre,
b) en présence d'un produit contenant des motifs oxyde d'alkylène,
c) en présence d'un produit polaire, l'eau étant exclue.

La réaction de l'invention se fait selon la formule : 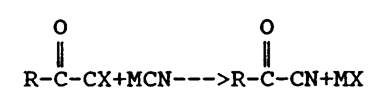
dans laquelle M désigne un métal alcalin. Après réaction, l'halogénure alcalin et l'excès éventuel de cyanure alcalin sont éliminés par filtration et lavage avec du solvant. Le cyanure d'acyle pur est obtenu par distillation du milieu réactionnel filtré.

Les halogénures d'acide utilisés comme matières premières sont définis par la formule (II). Dans cette formule R désigne de préférence un radical alkyle linéaire ou ramifié ayant de 1 à 4 carbones et pouvant être substitués ;
R désigne aussi, de préférence, un radical cycloalkyle ayant 5 ou 6 carbones et pouvant être substitué ;
R désigne aussi, de préférence, un radical phényle ou naphtyle et pouvant être substitué ;
R désigne aussi, de préférence, des restes hétérocycliques pentagonaux ou hexagonaux le cas échéant substitués.

Dans la formule (II), X désigne du chlore ou du brome.

Le cyanure alcalin, de préférence de sodium ou potassium, est utilisé en quantité stoéchiométrique ou bien en excès, à raison de 1 à 2 moles par mole d'halogénure d'acide, la quantité préférée est de 1 à 1,25 mole par mole d'halogénure d'acide.

L'halogénure d'acide est en général ajouté peu à peu au mélange réactionnel. Il est ajouté soit pur, soit dilué par du solvant de la réaction. La durée de l'addition peut varier de quelques minutes à plusieurs heures.

La durée préférée est d'environ 0,5 heure.

La réaction a lieu en présence d'un solvant en quantité suffisante pour permettre une bonne agitation des réactifs solides et liquides

On peut utiliser tous les solvants ne réagissant pas dans les conditions de la réaction avec l'halogénure d'acide, ou bien avec le cyanure alcalin. Les solvants très peu polaires sont préférés.

Comme solvant convenant à la réaction on peut citer :
- les hydrocarbures aromatiques tels que le benzène, le toluène, le xylène, etc ... et les hydrocarbures aromatiques halogénés tels que le chlorobenzène, le dichlorobenzène, etc...
- les hydrocarbures aliphatiques comme le cyclohexane, la ligroïne, etc...
- les hydrocarbures aliphatiques halogénés tels que le trichloroéthylène, le tétrachloroéthane, etc...

Les solvants préférés de la réaction sont le toluène et le xylène.

La quantité de solvant peut varier dans de larges limites. Avantageusement il suffit de 200 à 500 ml par mole d'halogénure d'acide.

On ne sortirait pas du cadre de l'invention en utilisant une quantité plus importante de solvant, mais on serait obligé de distiller une quantité plus importante pour récupérer le cyanure d'acyle.

La réaction peut s'effectuer à des températures comprises entre 60 et 150°C. La température préférée étant comprise entre 80 et 120°C.

La réaction s'effectue avantageusement à pression atmosphérique ou bien sous pression de vapeur du solvant, ou bien sous pression de gaz inerte.

La réaction est généralement achevée en 2 heures.

Cependant, il est avantageux de maintenir le chauffage pendant une durée de 2 à 8 heures afin d'éliminer toute trace de chlorure de benzoyle. Par exemple à 95°C la durée de réaction préférée est de 5 à 7 heures.

Le produit contenant des motifs oxyde d'alkylène contient avantageusement, il entre 2 et 200 motifs choisis parmi l'oxyde d'éthylène et l'oxyde de propylène.

C'est par exemple un produit contenant une ou plusieurs chaînes polyoxyéthylènes de formule :

-O-(CH₂-CH₂-O)ₙ-CH₂-CH₂-OH

où la somme du ou des n est comprise entre 2 et 200.

Les motifs oxyde d'éthylène pouvant être remplacés par des motifs oxyde de propylène ou un mélange de ces deux motifs.

Parmi les dérivés polyoxyalkylénés convenant bien, on peut citer :
- les polyéthylèneglycols simples de formule :

   OH-(CH₂-CH₂O)ₙ-CH₂-CH₂OH

   de masse moléculaire comprise entre 100 et 4000.
- les polyéthylèneglycols condensés sur l'acide stéarique de formule :

   CH₃-(CH₂)₁₆-COO-(CH₂-CH₂O)ₙ-CH₂-CH₂-OH

   où n est compris entre 20 et 150.
- les dérivés triglycérides des polyéthylèneglycols
- les alkylphénolpolyoxyéthylénés de formule : 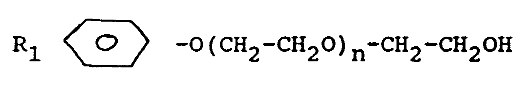
où n est compris entre 10 et 200 et R₁ désigne un alkyle ayant jusqu'à 20 carbones.

La liste non limitative comprend tous les dérivés du polyéthylèneglycol ayant plus de 10 motifs éthylène.

On peut utiliser les produits similaires aux produits ci-dessus dans lesquels le motif oxyde d'éthylène est remplacé par le motif oxyde de propylène ou un mélange de motifs oxyde d'éthylène-oxyde de propylène.

La quantité de ce produit peut varier de 0,1 à 10 g par mole d'halogénure d'acide. La quantité préférée étant de 0,4 à 2 g.

Le produit est généralement ajouté dans le solvant de la réaction mais il peut aussi être ajouté tout ou en partie avec l'halogénure d'acide pur ou dilué avec du solvant.

Lorsque la réaction a lieu en milieu rigoureusement anhydre avec des réactifs soigneusement deshydratés, le taux de conversion et le rendement sont très faibles, il est nécessaire d'ajouter aux réactifs une très petite quantité d'un produit polaire, tel que le formamide, le glycérol, l'éthylèneglycol pour obtenir un rendement et un taux de conversion élevés.

Le produit polaire est de préférence peu soluble dans le solvant de réaction mais ne doit pas donner lieu à la formation d'anhydride ou d'acide par réaction avec l'halogénure d'acide.

On utilise avantageusement les produits dont la constante diélectrique est supérieure à 30, sauf l'eau.

Parmi les dérivés polaires convenant particulièrement bien, on peut citer :
- le glycérol CH₂OH-CHOH-CH₂OH
- l'éthylène glycol CH₂OH-CH₂OH
- le formamide et les dérivés des sucres.

La quantité du produit polaire à ajouter est comprise entre 0,1 g et 10 g pour 1 mole d'halogénure d'acide.

La quantité préférée est comprise entre 0,25 et 2 g par mole d'halogénure d'acide.

Le mode d'introduction du produit polaire doit assurer une bonne répartition de celui-ci sur les réactifs.

Après réaction, l'excès éventuel de cyanure et d'halogénure alcalin formé sont éliminés par filtration. Le cyanure d'acyle pur est obtenu par distillation du milieu réactionnel filtré.

Dans les exemples suivants le taux de conversion exprime la quantité de l'halogénure d'acide disparue par rapport à la quantité initiale et le rendement exprime le rapport entre le nombre de moles de cyanure d'acyle obtenu et le nombre de moles d'halogénure d'acide initiales.

Dans les exemples la composition du filtrat est donnée en moles par rapport au chlorure de benzoyle engagé.

### EXEMPLE 1

Dans un réacteur en verre, muni d'une agitation et d'un réfrigérant contenant 150 cm³ de xylène et 0,5 g de nonylphénolpolyoxyéthyléné fourni par la Société GAF sous le nom ANTAROX CO 990, on ajoute 29,4 g de cyanure de sodium anhydre (0,6 mole) et 0,5 g de glycérol. On chauffe sous agitation à 95°C puis on coule en 0,5 heure 70,3 g de chlorure de benzoyle (0,5 mole), on maintient la température à 95°C pendant 6 heures puis, après refroidissement, on filtre et on lave avec du xylène ; on obtient un précipité minéral d'un poids de 34,5 g et un filtrat de 241,9 g.

Le filtrat organique est analysé par chromatographie en phase gaz et dosé par étalon interne.

| Composition molaire du filtrat : | |
|---|---|
| Chlorure de benzoyle | 0,4 % |
| Cyanure de benzoyle | 93,3 % |
| Anhydride benzoïque | 0,35 % |
| Acide benzoïque | 0,0 % |
| Dimère | 2,6 % |

Soit un rendement de 93,3 % et un taux de conversion du chlorure de benzoyle de 99,6 %.

### EXEMPLE 2

On procède exactement comme pour l'exemple 1 mais en remplaçant le glycérol par un poids égal d'éthylène glycol.

On obtient 229,1 g de filtrat organique contenant en % molaire par rapport au chlorure de benzoyle initial :

| | |
|---|---|
| Chlorure de benzoyle | 0,2 % |
| Cyanure de benzoyle | 91,6% |
| Anhydride benzoïque | 0,45 % |
| Acide benzoïque | 0,2 % |
| Dimère | 3,1% |

Soit un rendement de 91,6% et un taux de conversion de 99,8 %.

### EXEMPLE 3

On procède comme pour l'exemple 1 mais en remplaçant le glycérol par 1,25 de formamide. Après avoir coulé le chlorure de benzoyle en 1 heure, puis maintenu le chauffage pendant 2 heures. On obtient un précipité minéral de 33,4 g et un filtrat organique de 274,5 g contenant :

| | |
|---|---|
| Chlorure de benzoyle | 3,3 % |
| Cyanure de benzoyle | 76,0 % |
| Anhydride benzoïque | 1,0 % |
| Dimère | 10,0 % |

Soit un rendement de 76 % et un taux de conversion de 96,7 %.

### EXEMPLE 4

On procède exactement comme pour l'exemple 1, mais en remplaçant le glycérol par 0,5 g de glucose. On obtient un taux de conversion de 92 %, un rendement de 73,4 % et une teneur en anhydride benzoïque de 0,65 % (molaire) dans le filtrat.

### EXEMPLE 5

On procède exactement comme dans l'exemple 1, mais en diminuant la quantité de solvant : 75 cm³ de xylène au lieu de 150 cm³. On obtient un précipité minéral de 34,4 g et un filtrat organique de 182,2 g contenant :

| | |
|---|---|
| Benzonitrile | 0,1 % |
| Chlorure de benzoyle | 14,8 % |
| Cyanure de benzoyle | 79,0 % |
| Anhydride benzoïque | 0,8 % |
| Dimère | 3,2 % |

Soit un rendement de 79 % et un taux de transformation de 85,2 %.

### EXEMPLE 6 (non conforme à l'invention)

On procède exactement comme pour l'exemple 2 mais en omettant d'ajouter l'ANTAROX CO 990.

On obtient un précipité minéral de 36,6 g et un filtrat organique de 243,4 g, contenant :

| | |
|---|---|
| Chlorure de benzoyle | 57,8 % |
| Cyanure de benzoyle | 39,5 % |
| Acide benzoïque | 0,2 % |
| Anhydride benzoïque | 0,8 % |
| Dimère | 2,5 % |

Soit un rendement de 39,5 % et un taux de conversion de 42,2 %.

### EXEMPLE 7 (non conforme à l'invention)

On procède exactement comme pour l'exemple 1 mais en omettant d'ajouter le dérivé polaire. On obtient un précipité minéral de 30,8 g et un filtrat organique de 229 g contenant (% molaire) :

| | |
|---|---|
| Chlorure de benzoyle | 68,2 % |
| Cyanure de benzoyle | 23,8 % |
| Acide benzoïque | 0,3 % |
| Anhydride benzoïque | 0,5 % |

### EXEMPLE 8

On procède exactement comme pour l'exemple 1, mais en remplaçant l'ANTAROX CO 990 par le même poids de triglycéride de polyéthylèneglycol contenant 150 motifs éthylénés commercialisé par la Société ATLAS sous le nom de G 1295.

On obtient un précipité minéral de 34,9 g et un filtrat organique contenant en % molaire par rapport au chlorure de benzoyle initial :

| | |
|---|---|
| Benzonitrile | 0,2 % |
| Chlorure de benzoyle | 1,9 % |
| Cyanure de benzoyle | 92,3 % |
| Anhydride benzoïque | 0,45 % |
| Dimère | 2,5 % |

Soit un rendement chimique de 92,3 % pour un taux de conversion de 98,1 %

## Revendications

1. Procédé de synthèse de cyanures d'acyle de formule (I) : 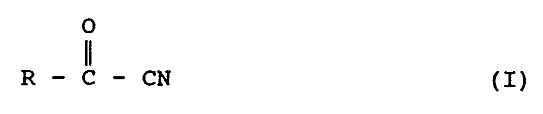 dans laquelle R est soit un radical alkyle ayant de 1 à 8 atomes de carbone, soit un radical cycloalkyle ayant de 3 à 12 atomes de carbone, soit un radical aryle, soit un reste hétérocyclique pouvant être condensé avec un noyau benzénique, tous ces radicaux R étant éventuellement substitués, consistant à faire réagir des halogénures d'acides de formule (II) : 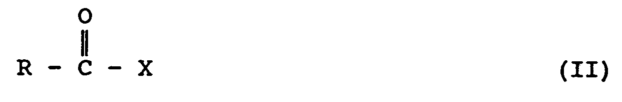 dans laquelle R a la définition précédente et X désigne le chlore ou le brome, avec des cyanures alcalins, caractérisé en ce que la réaction a lieu :
a) en milieu solvant anhydre,
b) en présence d'un produit contenant des motifs oxyde d'alkylène,
c) en présence d'un produit polaire, l'eau étant exclue.

2. Procédé selon la revendication 1 caractérisé en ce que le cyanure alcalin est utilisé à raison de 1 à 2 moles par mole d'halogénure d'acide et, de préférence, 1 à 1,25 mole.

3. Procédé selon l'une des revendications 1 à 2, caractérisé en ce qu'on utilise de préférence le cyanure de sodium.

4. Procédé selon l'une des revendications 1 à 3, caractérisé en ce qu'on utilise comme solvant, de préférence le xylène ou le toluène.

5. Procédé selon l'une des revendications 1 à 4, caractérisé en ce que le produit contenant des motifs oxyde d'alkylène contient entre 2 et 200 motifs choisis parmi l'oxyde d'éthylène ou l'oxyde de propylène.

6. Procédé selon la revendication 5, caractérise en ce que la quantité de produit est comprise entre 0,1 et 10 g, et de préférence, 0,4 à 2 g par mole d'halogénure d'acide.

7. Procédé selon l'une des revendications 1 à 6, caractérisé en ce que le produit polaire est choisi parmi le glycérol, l'éthylène glycol, le formamide et les dérivés des sucres.

8. Procédé selon l'une des revendications 1 à 7, caractérisé en ce que la quantité de produit polaire est comprise entre 0,1 et 10 g par mole d'halogénure d'acide, et de préférence, entre 0,25 et 2 g.

9. Procédé selon l'une des revendications 1 à 8, caractérisé en ce que l'halogénure d'acide est du chlorure de benzoyle.

10. Procédé selon l'une des revendications 1 à 9, caractérisé en ce que la quantité de solvant est comprise entre 200 et 500 ml par mole d'halogénure d'acide.

## Claims

1. Process for the synthesis of acyl cyanides of formula (I): 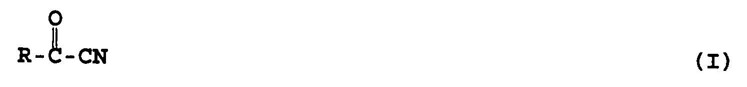 in which R is either an alkyl radical containing from 1 to 8 carbon atoms or a cycloalkyl radical containing from 3 to 12 carbon atoms, or an aryl radical, or a heterocyclic residue which may be condensed with a benzene nucleus, all these radicals R being optionally substituted, consisting in reacting acid halides of formula (II): 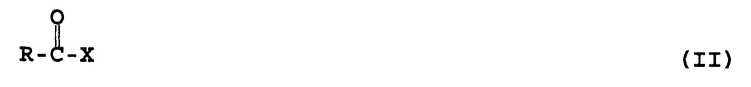 in which R has the above definition and X denotes chlorine or bromine, with alkali metal cyanides, characterized in that the reaction takes place:
a) in anhydrous solvent medium,
b) in the presence of a product containing alkylene oxide units,
c) in the presence of a polar product, water being excluded.

2. Process according to Claim 1, characterized in that the alkali metal cyanide is employed in a proportion of 1 to 2 moles, and preferably 1 to 1.25 moles, per mole of acid halide.

3. Process according to either of Claims 1 to 2, characterized in that sodium cyanide is preferably employed.

4. Process according to one of Claims 1 to 3, characterized in that xylene or toluene is preferably employed as solvent.

5. Process according to one of Claims 1 to 4, characterized in that the product containing alkylene oxide units contains between 2 and 200 units chosen from ethylene oxide and propylene oxide.

6. Process according to Claims 5, characterized in that the quantity of product is between 0.1 and 10 g, and preferably 0.4 to 2 g, per mole of acid halide.

7. Process according to one of Claims 1 to 6, characterized in that the polar product is chosen from glycerol, ethylene glycol, formamide and sugar derivatives.

8. Process according to one of Claims 1 to 7, characterized in that the quantity of polar product is between 0.1 and 10 g, and preferably between 0.25 and 2 g, per mole of acid halide.

9. Process according to one of Claims 1 to 8, characterized in that the acid halide is benzoyl chloride.

10. Process according to one of Claims 1 to 9, characterized in that the quantity of solvent is between 200 and 500 ml per mole of acid halide.

## Patentansprüche

1. Verfahren zur Herstellung von Acylcyaniden der Formel (1) 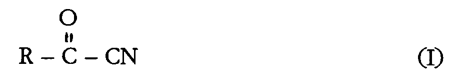 in der R ein Alkylrest mit 1 bis 8 Kohlenstoffatomen, ein Cycloalkylrest mit 3 bis 12 Kohlenstoffatomen, ein Arylrest, ein heterocyclischer Rest, gegebenenfalls mit kondensierten Benzolringen ist, wobei diese Reste R gegebenenfalls substituiert sein können, in dem man Säurehalogenide der Formel (II) 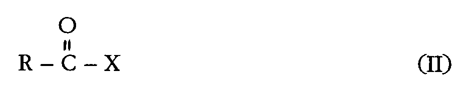 in der R der obigen Definition entspricht und X ein Chlor- oder Bromatom bezeichnet, mit Alkalicyaniden reagieren läßt, dadurch gekennzeichnet, daß die Reaktion
a) in einem wasserfreien Lösemittel
b) in Gegenwart eines Alkylenoxideinheiten enthaltenden Produktes,
c) in Gegenwart eines polaren Produktes, ausgenommen Wasser, durchgeführt wird.

2. Verfahren nach Anspruch 1, dadurch gekennzeichnet, daß 1 bis 2 Mol, vorzugsweise 1 bis 1,25 Mol Alkalicyanid pro Mol Säurehalogenid verwendet werden.

3. Verfahren nach einem der Ansprüche 1 bis 2, dadurch gekennzeichnet, daß man vorzugsweise Natriumcyanid verwendet.

4. Verfahren nach einem der Ansprüche 1 bis 3, dadurch gekennzeichnet, daß man als Lösemittel vorzugsweise Xylol oder Toluol verwendet.

5. Verfahren nach einem der Ansprüche 1 bis 4, dadurch gekennzeichnet, daß das Alkylenoxideinheiten enthaltende Produkt zwischen 2 und 200 Ethylenoxid- oder Propylenoxid-Einheiten enthält.

6. Verfahren nach Anspruch 5, dadurch gekennzeichnet, daß die Menge des Produkts zwischen 0,1 und 10 g, vorzugsweise zwischen 0,4 und 2 g pro Mol Säurehalogenid liegt.

7. Verfahren nach einem der Ansprüche 1 bis 6, dadurch gekennzeichnet, daß das polare Produkt aus der Gruppe von Glycerin, Ethylenglykol, Formamid und Zuckerderivaten gewählt ist.

8. Verfahren nach einem der Ansprüche 1 bis 7, dadurch gekennzeichnet, daß die Menge des polaren Produktes zwischen 0,1 und 10 g, vorzugsweise zwischen 0,25 und 2 g pro Mol Säurehalogenid beträgt.

9. Verfahren nach einem der Ansprüche 1 bis 8, dadurch gekennzeichnet, daß das Säurehalogenid Benzoylchlorid ist.

10. Verfahren nach einem der Ansprüche 1 bis 9, dadurch gekennzeichnet, daß die Menge an Lösemittel zwischen 200 und 500 ml pro Mol Säurehalogenid beträgt.
